Europäisches Patentamt

European Patent Office

Office européen des brevets

(19)

(11) Veröffentlichungsnummer : **0 356 475 B1**

# (12) EUROPÄISCHE PATENTSCHRIFT

(45) Veröffentlichungstag der Patentschrift :
**15.04.92 Patentblatt 92/16**

(51) Int. Cl.$^5$ : **A63C 9/088**

(21) Anmeldenummer : **89901858.4**

(22) Anmeldetag : **01.02.89**

(86) Internationale Anmeldenummer :
**PCT/EP89/00082**

(87) Internationale Veröffentlichungsnummer :
**WO 89/07476 24.08.89 Gazette 89/20**

(54) **SICHERHEITSSKIBINDUNG.**

(30) Priorität : **19.02.88 AT 405/88**

(43) Veröffentlichungstag der Anmeldung :
**07.03.90 Patentblatt 90/10**

(45) Bekanntmachung des Hinweises auf die
Patenterteilung :
**15.04.92 Patentblatt 92/16**

(84) Benannte Vertragsstaaten :
**CH DE FR LI**

(56) Entgegenhaltungen :
**EP-A- 0 042 762
WO-A-81/00358
WO-A-83/03555
AT-A- 315 037
US-A- 4 387 307**

(73) Patentinhaber : **TMC Corporation
Ruessenstrasse 16
CH-6340 Baar/Zug (HR Nr. 15127) (CH)**

(72) Erfinder : **PLATTER, Ferdinand
Feliusstr. 4
I-39023 Laas (IT)**
Erfinder : **ROSENICH, Paul
Simmeringer Hauptstr. 60-64
A-1110 Wien (AT)**

(74) Vertreter : **Szász, Tibor, Dipl.-Ing.
Schlossmühlstrasse 1
A-2320 Schwechat (AT)**

**Beschreibung**

Die Erfindung bezieht sich auf eine Sicherheitsskibindung mit mindestens einem die auf die Bindung einwirkenden Kräfte in elektrische Signale umwandelnden Wandler und einer mit einer Auslöseeinrichtung und dem Wandler verbundenen Auswerteschaltung, die eine mit einer Zeitschaltung verbundene Schwellwertschaltung aufweist, welche Zeitschaltung im wesentlichen durch einen in seiner Frequenz steuerbaren Oszillator und einen diesem nachgeschalteten Zähler samt Komparator gebildet ist, der bei Überschreiten eines vorgegebenen Wertes die Auslöseeinrichtung aktiviert, wobei der bzw. die Wandler mit dem Eingang der Schwellwertschaltung und dem Eingang der Zeitschaltung verbunden ist, bzw. sind.

Durch die WO 81/00 358-A1 wurde eine Sicherheitsskibindung der eingangs erwähnten Art bekannt, bei der die Meßwertaufnehmer über einen Multiplexer mit einem umschaltbaren Verstärker und einem Taktgeber verbunden sind. Der Verstärker steuert dabei einen spannungsgesteuerten Oszillator dessen Ausgangssignale in zugeordneten Zählern Zählern gezählt werden, die bei Überschreiten eines bestimmten Zählerstandes ein Freigabesignal an die Auslöseeinrichtung abgeben. Dabei werden Signale des spannungsgesteuerten Oszillators jeweils den den einzelnen Meßwertaufnehmern zugeordneten Zählern zugeleitet, wobei die entsprechende Umschaltung durch den Taktgeber erfolgt.

Damit wird zwar eine von der Größe der anstehenden Kräfte abhängige Auslösegeschwindigkeit erreicht, es ergibt sich allerdings der Nachteil, daß auch bei höchsten Belastungen eine gewisse Verzögerung in der Ansteuerung der Auslöseeinrichtung nicht zu vermeiden ist. So arbeitet der in seiner Frequenz gesteuerte Oszillator in einem solchen Falle aufgrund des hohen Signalpegels, den die Meßwertaufnehmer liefern, zwar mit einer entsprechend hohen Frequenz, doch kann die Ansteuerung der Auslöseeinrichtung erst beginnen, bis der vorgesehene Zählerstand, der für eine Auslösung festgelegt ist, erreicht ist. Dies kann aber in extremen Fällen zu einer verspäteten Auslösung und damit zu einer erheblichen Verletzungsgefahr für den Skifahrer führen.

Ziel der Erfindung ist es, diese Nachteile zu vermeiden und eine Sicherheitsskibindung der eingangs erwähnten Art vorzuschlagen, bei der die Auslösekennlinie an die Belastbarkeit des Benutzers anpaßbar ist und bei der bei entsprechend hohen, auf den Skifahrer bzw. die Bindung einwirkenden Kräften eine unverzögerte Auslösung möglich ist.

Erfindungsgemäß wird dies dadurch erreicht, daß die Schwellwertschaltung in an sich bekannter Weise mindestens zwei in Abhängigkeit von verschiedenen Schwellwerten gesteuerte Ausgänge aufweist, deren vom höchsten Schwellwert gesteuerter Ausgang die Auslöseeinrichtung unverzögert ansteuert und deren zweiter von einem niedrigeren Schwellwert gesteuerter Ausgang mit dem Zähler der Zeitschaltung verbunden ist.

Durch diese Maßnahmen wird erreicht, daß bei einem einen bestimmten, hohen Pegel des Ausgangssignales des Wandlers die Auslöseeinrichtung unverzögert angesteuert wird, wogegen bei Signalwerten des Wandlers, die zwischen einer niedrigeren, die Zeitschaltung aktivierenden Schwelle und der erwähnten obersten Schwelle liegen, die Auslöseeinrichtung mit einer, in Abhängkeit von der Größe des vom Meßwertaufnehmer gelieferten Signales variablen Zeitverzögerung aktiviert wird. Damit wird einerseits eine unverzügliche Auslösung der Bindung bei sehr hohen Belastungen und anderseits eine belastungsabhängig verzögerte Auslösung bei in einem bestimmten Bereich liegenden Belastungen sichergestellt, wodurch einerseits Fehlauslösungen vermieden werden und anderseits ein besonders hohes Maß an Sicherheit auch bei höchsten Belastungen gewährleistet ist.

In diesem Zusammenhang kann weiters vorgesehen sein, daß die Schwellwertschaltung mit einem Freigabeeingang des Zählers direkt und mit einem Rückstelleingang des Zählers über einen Inverter und gegebenenfalls einem diesem nachgeschalteten monostabilen Flip-Flop mit kurzer Ablaufzeit verbunden ist.

Damit ist sichergestellt, daß der Zähler nur dann aktiviert wird, wenn ein den einen Schwellwert übersteigendes Signal an der Schwellwertschaltung anliegt und eine Rückstellung des Zählers erfolgt, sobald dieser Pegel wieder unterschritten wird, wobei das Flip-Flop einen entsprechenden Nadelimpuls liefert.

Um besonders bei sehr kleinen und über eine längere Zeit anstehenden Ausgangssignalen des Wandlers bzw. der Wandler definierte Verhältnisse zu schaffen, kann nach einem weiteren Merkmal der Erfindung vorgesehen sein, daß der in seiner Frequenz variable Oszillator zwischen einer von Null verschiedenen Frequenz und einer maximalen Frequenz veränderbar ist.

Damit wird sichergestellt, daß bei knapp über der Aktivierungsschwelle des Zählers liegenden Belastungen eine Auslösung der Bindung innerhalb einer bestimmten, durch die Mindestfrequenz des Oszillators im wesentlichen festgelegten Zeit erfolgt und dadurch eine Gefährdung der Gesundheit des Benutzers durch hohe Belastungen über eine größere Zeitspanne sicher vermieden werden.

In diesem Zusammenbang kann nach einem weiteren Merkmal der Erfindung vorgesehen sein, daß der in seiner Frequenz variable Oszillator als spannungsgesteuerter Oszillator ausgebildet ist, der mit einer der Mindestfrequenz entsprechenden, einstellbaren Spannung beaufschlagt ist.

Dadurch kann der der Oszillator durch die im allgemeinen als Spannungen vorliegenden Ausgangssignale des Wandlers bzw. der Wandler unmittelbar gesteuert werden.

Nach einem weiteren Merkmal der Erfindung kann vorgesehen sein, daß der mit dem Zähler der Zeitschaltung verbundene Ausgang der Schwellwertschaltung weiters mit einem Eingang eines UND-Gatters verbunden ist, dessen zweiter Eingang mit dem Ausgangt des dem Zähler der Zeitschaltung nachgeschalteten Komparators verbunden ist, wobei der Ausgang des UND-Gatters mit der Auslöseeinrichtungt verbunden ist.

Durch diese Maßnahmen ergibt sich der Vorteil, daß eine Auslösung nur dann erfolgt, wenn der für eine Auslösung erforderliche Zählerstand der Zeitschaltung erreicht ist und das Ausgangssignal des Wandlers noch einen über den für eine Aktivierung der Zeitschaltung vorgesehenen Schwellenpegel der Schwellwertschaltung liegenden Pegel aufweist. Damit wird vermiden, daß es im Zeitpunkt des Abfalles der Belastung unter einen unkritischen Wert noch zu einer Auslösung kommen kann, wodurch Fehlauslösungen unterbleiben.

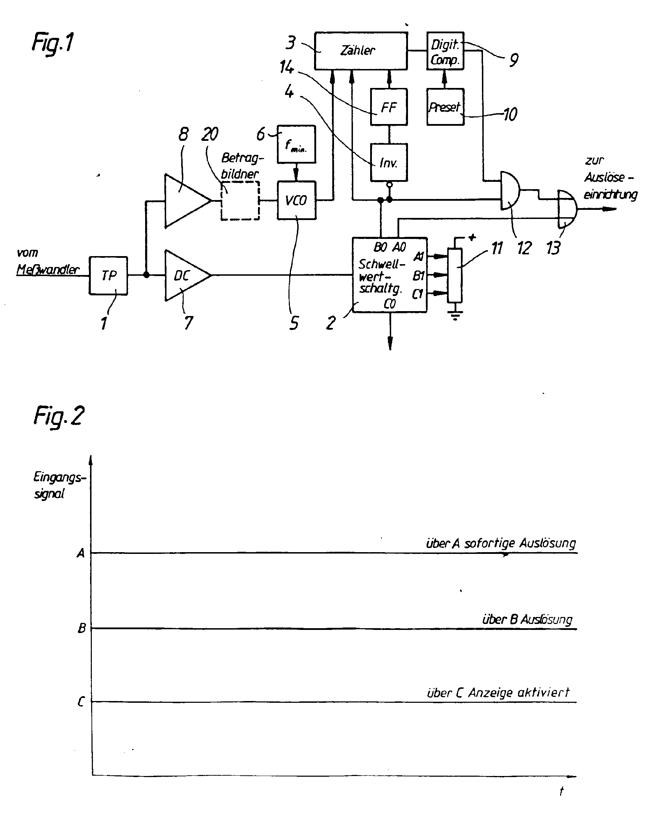

Die Erfindung wird nun anhand der Zeichnung näher erläutert. Dabei zeigt Fig. 1 schematisch eine Ausverteschaltung für eine erfindungsgemäße Skibindung und Fig. 2 ein Diegramm der Schwellwerte.

Der Meßsignaleingang ist mit nicht dargestellten Wandlern verbunden, die die auf die Bindung einwirkenden Kräfte in entsprechende elektriche Signale umwandeln und z. B: durch Dehnungsmeßstreifen oder Piezoelemente od. dgl. gebildet sein können. Diese Signale gelangen über ein Tiefpassfilter 7, das zur Ausfilterung von durch Schwingungen der Skier herrührenden höherfrequenten Schwankungen des Meßsignales dienen und einen gleichstromgekoppelten Verstärker 1 zu einer Schwellwertschaltung 2, die die einlangenden Signale nach drei Schwellwerten Klassiert und an den entsprechenden Ausgängen $A_0$, $B_0$ und $C_0$ Signale liefert, sobald das Eingangssignal die entsprechenden, über die Stelleingänge $A_1$, $B_1$ und $C_1$ einstellbaren Schwellwerte überschritten hat.

Aus der Fig. 2 ist dabei zu ersehen, daß der Schwellwert A den höchsten, Schwellwert B den mittleren und der Schwellwert C den niedrigsten Pegel aufweist. Je nach dem welchen Pegel das vom Wandler kommende Signal aufweist, erscheint an keinem der Ausgänge $A_0$ bis $C_0$ ein Signal, oder an einem bis drei der Ausgänge. Ersteres ist der Fall, wenn der Pegel C nicht überschritten wird und letzteres, wenn der Pegel A überschritten wird.

Damit wird sichergestellt, daß bei knapp über der Aktivierungsschwelle des Zählers liegenden Belastungen eine Auslösung der Bindung innerhalb einer bestimmten, durch die Mindestfrequenz des Oszillators im wesentlichen festgelegten Zeit erfolgt und dadurch eine Gefährdung der Gesundheit des Benutzers durch hohe Belastungen über eine größere Zeitspabbe sicher vermieden werden.

In diesem Zusammenhang kann nach einem weiteren Merkmal der Erfindung vorgesehen sein, daß der in seiner Frequenz variable Oszillator als spannungsgesteuerter Oszillator ausgebildet ist, der mit einer der Mindestfrequenz entsprechenden, einstellbaren Spannung beaufschlagt ist.

Weiters ist es vorteilhaft, wenn ein oberer Schwellwert einstellbar ist, bei dessen Überschreiten die Auslöseeinrichtung unverzüglich aktivierbar ist.

Die Erfindung wird nun anhand der Zeichnung näher erläutert. Dabei zeigt:

Fig. 1 schematisch eine Auswerteschaltung für eine erfindungsgemäße Skibindung,

Fig. 2 ein Diagramm der Schwellwerte.

Der Meßsignaleingang ist mit nicht dargestellten Wandlern verbunden, die die auf die Bindung einwirkenden Kräfte in entsprechende elektrische Signale umwandeln und z.B: durch Dehnungsmeßstreifen oder Piezoelemente od. dgl. gebildet sein können. Diese Signale gelangen über ein Tiefpassfilter 7, das zur Ausfilterung von durch Schwingungen der Skier herrührenden höherfrequenten Schwankungen des Meßsignales dienen und einen gleichstromgekoppelten Verstärker 1 zu einer Schwellwertschaltung 2, die die einlangenden Signale nach drei Schwellwerten klassiert und an den entsprechenden Ausgängen $A_0$, $B_0$ und $C_0$ Signale liefert, sobald das Eingangssignal die entsprechenden, über die Stelleingänge $A_1$, $B_1$ und $C_1$ einstellbaren Schwellwerte überschritten hat.

Aus der Fig. 2 ist dabei zu ersehen, daß der Schwellwert A den höchsten, Schwellwert B den mittleren und der Schwellwert C den niedrigsten Pegel aufweist. Je nach dem welchen Pegel das vom Wandler kommende Signal aufweist, erscheint an keinem der Ausgänge $A_0$ bis $C_0$ ein Signal, oder an einem bis drei der Ausgänge. Ersteres ist der Fall, wenn der Pegel C nicht überschritten wird und letzteres, wenn der Pegel A überschritten wird.

Die einem Überschreiten eines jeden der vorbestimmten Pegel der Eingangssignale zugeordneten Ausgangsignale der Schwellwertschaltung 2 erscheinen an getrennten Ausgängen, wobei der Ausgang $C_0$ z.B. mit einer Anzeige verbunden sein kann, an der erkennbar ist, ob der Skischuh korrekt in der Bindung gehalten ist, oder z.B. durch eine Schneeauflage ein unkorrekter Sitz gegeben ist.

Der Ausgang $B_0$ liefert ein Signal, wenn ein Eingangssignal ansteht, das einer Belastung entspricht die nur für eine relativ kurze Zeit toleriert werden kann. Dieser Ausgang ist direkt mit einem Freigabeeingang und über einen Inverter 4 und einem diesem nachfeschalteten monostabilen Flip-Flop 14, das eine kurze Laufzeit

3

aufweist und zur Erzeugung eines Nadelimpulses dient, mit einem Rücksetzeingang eines Zählers 3 verbunden. Dieser erhält seine Zählimpulse von einem in seiner Frequenz steuerbaren oszillator, der vorzugsweise als spannungsgesteuerter Oszillator 5 ausgebildet ist. Allerdings kann der Zähler die Impulse des Oszillators nur zählen, solange ein Freigabesignal vom Ausgang $B_0$ der Schwellwertschaltung 2 am Steuereingang des Zählers 3 anliegt. Verschwindet ein solches Signal vom Ausgang $B_0$ so gibt der Inverter 4 ein Signal an seinem Ausgang ab und startet den monostabilen Flip-Flop 14, der, bedingt durch dessen kurze Laufzeit, einen Nadelimpuls abgibt, der den Zähler 3 zurückstellt. Damit ist sichergestellt, daß der Zähler bis zum abermaligen Eintreffen eines Signales am Ausgang $B_0$ der Schwellwertschaltung 2 rückgestellt ist und sofort mit der Zählung beginnen kann.

Gesteuert wird dieser Oszillator 5 von einem Sollwertgeber 6 für eine bestimmte Mindestfrequenz, der durch eine einstellbare Spannungsquelle gegeben sein kann, und dem Ausgangssignal eines über Filter 7 mit dem meßsignaleingang verbundenen Verstärkers 8, der eine variable Verstärkung aufweist und z.B. mit einem Dioden aufweisenden Rückkopplungsnetzwerk versehen ist. Dabei kann vorgesehen sein, daß dieser Verstärker 8 mit verschiedenen Rückkopplungsnetzwerken bestückbar ist. Dies ermöglicht eine Anpassung der Auslösekennlinie an die Bedürfnisse des Benutzers.

Falls die Meßwandler positive und negative Signale abgeben, kann zwischen dem Verstärker 8 und dem spannungsgesteuerten Oszillator 5 ein Betragbildner 20 zwischengeschaltet sein, der an seinem Ausgang stets nur Spannung einer Polarität z.B. positiv gegen ein Bezugspotential, abgibt, unabhängig von der Polarität seiner Eingangsspannung gegen dieses Bezugspotential.

Der Verstärker 8 liefert ein Ausgangssignal in Abhängigkeit von dem von den nicht dargestellten Wandlern gelieferten Eingangssignal. Dadurch wird erreicht, daß, sobald das Eingangssignal den Pegel B überschreitet und daher der Zähler 3 freigegeben wird, der Zähler 3 mit Zählimpulsen in Abhängigkeit von der Amplitude des Eingangssignales oder dessen Anstieg, beaufschlagt wird und daher mehr oder weniger rasch seinen Zählerstand erhöht.

Dieser Zählerstand wird laudend von dem mit dem Ausgang des Zählers 3 verbundenen Komparator 9 mit einem vorgegebenen Wert verglichen, der über eine Eingabeeinrichtung 10 dem Komparator 9 eingegeben werden kann. Diese Eingabeeinrichtung ist zweckmäßigerweise derart angeordnet, daß sie nur einem sachkundigen Personal zugänglich ist, desgleichen die Einstelleinrichtung 11 zur Festlegung der Schwellwerte der Schwellwerteinrichtung 2.

Der Komparator 9 gibt bei einem Gleichstand des Zählerstandes mit dem vorgegebenen Vergleichswert ein Signal ab das über ein UND-Glied 12, dessen zweiter Eingang mit dem Ausgang $B_0$ der Schwellwertschaltung 2 verbunden ist und ein ODER-Glied 13 der nicht dargestellten Auslöseeinrichtung zugeführt wird. Der zweite Eingang des ODER-Gliedes 13 ist dabei mit dem Ausgang $A_0$ der Schwellwertschaltung 2 verbunden, sodaß, sobald der Pegel A des Eingangssignales überschritten ist, die Auslöseeinrichtungt unverzögert angesteuert wird.

Wird dagegen nur der Pegel B durch das Eingangssignal überschritten, so erfolgt eine Ansteuerung der Auslöseeinrichtung mit einer Verzögerung, die beim dargestellten Ausführungsbeispiel vom Betrag der Amplitude des Eingangssignales abhängt, oder, falls das Eingangssignal vor dem Erreichen der entsprechenden Verzögerungszeit wieder unter den Pegel B fällt, überhaupt nicht.

Auf diese Weise werden Auslösungen der Bindung bei zwar relativ hohen, aber nur ungefährlich lange andauernden Belastungen vermieden.

Bezüglich des UND-Gliedes 12 ist zu bemerken, daß auf dieses auch verzichtet werden kann, und der Ausgang des Komparators 9 direkt mit einem Eingang des ODER-Gliedes 13 verbunden werden kann.

Die Anordnung eines Sollwertgebers 6 für die Mindestfrequenz des Oszillators 5 ermöglicht es, die maximale Zeit bis zu einer Ansteuerung der Auslöseeinrichtung bei nur knapp über dem Pegel B liegenden, bzw. sich nur langsam ändernden Eingangssignalen, die über längere Zeit anstehen, entsprechend zu begrenzen.

Beim dargestellten Ausführungsbeispiel sind drei Schwellwerte vorgesehen, doch ist deren Anzahl grundsätzlich unbeschränkt, wobei mit steigender Anzahl der vorgesehenen Pegel eine bessere Anpassung an die anatomischen Gegebenheiten des Benutzers ermöglich wird.

## Patentansprüche

1. Sicherheitsskibindung mit mindestens einem die auf die Bindung einwirkenden Kräfte in elektrische Signale umwandelnden Wandler und einer mit einer Auslöseeinrichtung und dem Wandler verbundenen Auswerteschaltung, die eine mit einer Zeitschaltung verbundene Schwellwertschaltung aufweist, welche Zeitschaltung im wesentlichen durch einen in seiner Frequenz steuerbaren Oszillator und einen diesem nachgeschalteten Zähler samt Komparator gebildet ist, der bei Überschreiten eines vorgegebenen Wertes die Auslöseeinrichtung aktiviert, wobei der bzw. die Wandler mit dem Eingang der Schwellwertschaltung und dem Eingang der Zeitschaltung verbunden ist, bzw. sind, dadurch gekennzeichnet, daß die Schwellwertschaltung (2) in an sich bekannter Weise mindestens zwei in Abhängigkeit von verschiedenen Schwellwerten gesteuerte Ausgänge ($A_0$, $B_0$, $C_0$) aufweist, deren vom höchsten Schwellwert gesteuerter Ausgang ($A_0$) die Auslöseeinrichtung unverzögert ansteuert und deren zweiter von einem niedrigeren Schwellwert gesteuerter Ausgang ($B_0$) mit dem Zähler (3) der Zeitschaltung verbunden ist.

2. Sicherheitsskibindung nach Anspruch 1, dadurch gekennzeichnet, daß die Schwellwertschaltung (2) mit einem Freigabeeingang des Zählers (3) direkt und mit einem Rückstelleingang des Zählers (3) über einen Inverter (4) und gegebenenfalls einem diesem nachgeschalteten monostabilen Flip-Flop (14) mit kurzer Laufzeit verbunden ist.

3. Sicherheitsskibindung nach Anspruch 1 oder 2, dadurch gekennzeichnet, daß der in seiner Frequenz variable Oszillator (5) zwischen einer von Null verschiedenen Frequenz und einer maximalen Frequenz veränderbar ist.

4. Sicherheitsskibindung nach einem der Ansprüche 1 bis 3, dadurch gekennzeichnet, daß der in seiner Frequenz variable Oszillator (5) als spannungsgesteuerter Oszillator ausgebildet ist, der mit einer der Mindestfrequenz entsprechenden, einstellbaren Spannung beaufschlagt ist.

5. Sicherheitsskibindung nach einem der Ansprüche 1 bis 4, dadurch gekennzeichnet, daß der mit dem Zähler (3) der Zeitschaltung verbundene Ausgang ($B_0$) der Schwellwertschaltung (2) weiters mit Eingang eines UND-Gatters (12) verbunden ist, dessen zweiter Eingang mit dem Ausgang des dem Zähler (3) der Zeitschaltung nachgeschalteten Komparators (9) verbunden ist, wobei der Ausgang des UND-Gatters (12) mit der Auslöseeinrichtung verbunden ist.

## Revendications

1. Fixation de sécurité pour skis, comprenant au moins un convertisseur convertissant en des signaux électriques les forces agissant sur la fixation, ainsi qu'un circuit d'interprétation qui est raccordé à un dispositif de déclenchement et au convertisseur, et présente un circuit à valeurs de seuil raccordé à un circuit de temporisation, lequel circuit de temporisation est formé, pour l'essentiel, d'un oscillateur à fréquence commandable et d'un compteur branché en aval de ce dernier et associé à un comparateur, qui active le dispositif de déclenchement lors du dépassement d'une valeur préétablie, le(s) convertisseur(s) étant raccordé(s) à l'entrée du circuit à valeurs de seuil et à l'entrée du circuit de temporisation, caractérisée par le fait que le circuit (2) à valeurs de seuil présente, d'une manière connue par elle-même, au moins deux sorties ($A_0$, $B_0$, $C_0$) commandées en fonction de différentes valeurs de seuil, parmi lesquelles la sortie ($A_0$), commandée par la valeur de seuil la plus forte, active instantanément le dispositif de déclenchement, et la deuxième sortie ($B_0$), commandée par une valeur de seuil moindre, est raccordée au compteur (3) du circuit de temporisation.

2. Fixation de sécurité pour skis selon la revendication 1, caractérisé par le fait que le circuit (2) à valeurs de seuil est raccordé, directement, à une entrée de validation du compteur (3) et par l'intermédiaire d'un inverseur (4) et éventuellement d'une bascule monostable (14) à courte durée d'action branchée en aval dudit inverseur, à une entrée de remise à zéro de ce compteur (3).

3. Fixation de sécurité pour skis selon la revendication 1 ou 2, caractérisée par le fait que l'oscillateur (5) à fréquence variable est modulable entre une fréquence différant de zéro et une fréquence maximale.

4. Fixation de sécurité pour skis selon l'une des revendications 1 à 3, caractérisée par le fait que l'oscillateur (5) à fréquence variable est réalisé sous la forme d'un oscillateur commandé en tension, auquel est appliquée une tension réglable correspondant à la fréquence minimale.

5. Fixation de sécurité pour skis selon l'une des revendications 1 à 4, caractérisée par le fait que la sortie ($B_0$) du circuit (2) à valeurs de seuil, raccordée au compteur (3) du circuit de temporisation, est par ailleurs raccordée à une entrée d'une porte ET (12) dont la seconde entrée est raccordée à la sortie du comparateur (9) branché en aval du compteur (3) du circuit de temporisation, la sortie de la porte ET (12) étant raccordée au dispositif de déclenchement.

## Claims

1. A safety ski binding comprising at least one converter acting to convert the forces applied to said binding into electric signals, and an evaluation circuit connected to a release device and to said converter and including a threshold value circuit connected to a timer circuit substantially composed of a frequency-controllable oscillator and at the output side thereof a counter in combination with a comparator operable to activate said release device in response to a preselected value being exceeded, said converter, or converters, respectively, being connected to the input of said threshold value circuit and the input of said timer circuit, characterized in that said threshold value circuit (2) is provided in a per se known manner with at least two outputs (Ao, Bo, Co) controlled in response to different threshold values, the output (Ao) controlled by the highest threshold value acting to activate said release device without delay, and the second output (Bo) controlled by a lower threshold value being connected to said counter (3) of said timer circuit.

2. A safety ski binding according to claim 1, characterized in that said threshold value circuit (2) is connected directly to a release input of said counter (3), and indirectly to a reset input of said counter (3) by way of an inverter (4) and, optionally, a monostable multivibrator (14) of short rise time connected thereto at its output side.

3. A safety ski binding according to claim 1 or 2, characterized in that said frequency-controllable oscillator (5) is controllable between a frequency other than Zero and a maximum frequency.

4. A safety ski binding accordingt to any of claims 1 to 3, characterized in that said frequency-controllable oscillator (5) is devised as a voltage-controlled oscillator having an adjustable voltage applied thereto corresponding to its minimum frequency.

5. A safety ski binding accordint to any of claims 1 to 4, characterized in that said output (Bo) of said threshold value circuit (2) connected to said counter (3) of said timer circuit is additionally connected to an input of an AND gate (12) having its second input connected to the output of said comparator (9) controlled by the output of said counter (3) of said timer circuit, the output of said AND gate (12) being connected to said release device.

**Fig.1**

**Fig.2**